(19) Europäisches Patentamt
European Patent Office
Office européen des brevets

(11) Veröffentlichungsnummer: **0 053 311**
**B1**

(12) # EUROPÄISCHE PATENTSCHRIFT

(45) Veröffentlichungstag der Patentschrift: **04.09.85**

(51) Int. Cl.⁴: **C 07 D 249/08**, A 01 N 43/64

(21) Anmeldenummer: **81109686.6**

(22) Anmeldetag: **14.11.81**

(54) **Substituierte 1-Phenyl-2-triazolyl-1-penten-3-ole, Verfahren zu ihrer Herstellung sowie ihre Verwendung als Pflanzenwachstumsregulatoren und Fungizide.**

(30) Priorität: **28.11.80 DE 3044802**

(43) Veröffentlichungstag der Anmeldung: **09.06.82 Patentblatt 82/23**

(45) Bekanntmachung des Hinweises auf die Patenterteilung: **04.09.85 Patentblatt 85/36**

(84) Benannte Vertragsstaaten: **AT BE CH DE FR GB IT LI NL SE**

(56) Entgegenhaltungen:
**EP - A - 0 015 387**
**EP - A - 0 022 975**
**EP - A - 0 028 363**
**DE - A - 2 920 437**
**FR - A - 2 327 242**
**GB - A - 2 004 276**
**GB - A - 2 046 260**
**US - A - 4 182 862**

(73) Patentinhaber: **BAYER AG, Konzernverwaltung RP Patentabteilung, D-5090 Leverkusen 1 Bayerwerk (DE)**

(72) Erfinder: **Regel, Erik, Bergerheide 72a, D-5600 Wuppertal 1 (DE)**
Erfinder: **Büchel, Karl-Heinz, Prof. Dr., Dabringhausener Strasse 42, D-5093 Burscheid (DE)**
Erfinder: **Lürssen, Klaus, Dr., August-Kierspel-Strasse 89, D-5070 Bergisch Gladbach (DE)**
Erfinder: **Frohberger, Paul-Ernst, Dr., Willi-Baumeister-Strasse 5, D-5090 Leverkusen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft neue substituierte 1-Phenyl-2-triazolyl-1-penten-3-ole, ein Verfahren zu ihrer Herstellung sowie ihre Verwendung als Pflanzenwachstumsregulatoren und Fungizide.

Es ist bereits bekannt geworden, dass bestimmte 4,4-Dimethyl-1-phenyl-2-triazolyl-1-penten-3-ole eine gute fungizide Wirksamkeit besitzen (vgl. DE-A-2 838 847 und GB-A-2 004 276). Die Wirkung dieser Verbindungen ist jedoch, insbesondere bei niedrigen Aufwandmengen und -konzentrationen, nicht immer voll befriedigend. Die pflanzenwachstumsregulierende Wirkung dieser Azol-Derivate ist ebenfalls nicht immer ganz befriedigend.

Weiterhin sind aus der GB-A-2 046 260 geometrische Isomere von bestimmten 1-Triazolylstyrol-Derivaten mit pflanzenwuchsregulierenden und fungiziden Eigenschaften bekannt. Es werden in dieser Druckschrift jedoch keine Verbindungen offenbart, in denen der Phenyl-Ring der Styrol-Einheit durch Halogenalkoxy, Halogenalkylthio, Hydroxy, Dialkylamino, Alkylcarbonyloxy oder Benzyloxy substituiert ist.

Es wurden nun neue substituierte 1-Phenyl-2-triazolyl-1-penten-3-ole der Formel

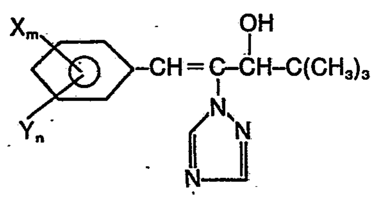

(I)

in welcher

X für Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Hydroxy, Dialkylamino mit 1 bis 4 Kohlenstoffatomen in jedem Alkylteil, Alkylcarbonyloxy mit 1 bis 4 Kohlenstoffatomen im Alkylteil oder für Benzyloxy steht,

Y für Fluor, Chlor, Brom, Alkyl mit 1 bis 4 Kohlenstoffatomen oder Alkoxy mit 1 bis 4 Kohlenstoffatomen steht,

m für 1 oder 2 steht und

n für 0, 1 oder 2 steht

sowie deren 1,5-Naphthalin-disulfonsäure- oder Chlorwasserstoff-Additionssalze gefunden.

Die erfindungsgemässen Verbindungen der Formel (I) kommen in den geometrischen Isomeren E (trans) und Z (cis) vor.

Bei der E, Z-Nomenklatur werden die an der Doppelbindung stehenden Sustituenten nach der Cahn-Ingold-Prelog-Regel nach abnehmender Priorität eingeordnet. Stehen die bevorzugten Substituenten auf derselben Seite der Doppelbindung, liegt die Konfiguration Z (abgeleitet von zusammen) vor, stehen sie auf der entgegengesetzten Seite, liegt die Konfiguration E (abgeleitet von entgegen) vor.

Da ausserdem ein asymmetrisches Kohlenstoffatom vorhanden ist, können die Verbindungen der Formel (I) in zwei optischen Isomerenformen vorkommen.

Die vorliegende Erfindung betrifft sowohl die einzelnen Isomeren als auch die Isomerengemische.

Weiterhin wurde gefunden, dass man die substituierten 1-Phenyl-2-triazolyl-1-penten-3-ole der Formel (I) erhält, wenn man 1-Phenyl-2-triazolyl-1-penten-3-one der Formel

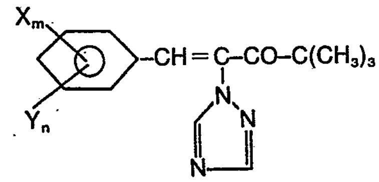

(II)

in welcher

X, Y, n und m die oben angegebene Bedeutung haben,

in allgemein üblicher Weise reduziert.

An die so erhaltenen Verbindungen der Formel (I) kann gegebenenfalls anschliessend Chlorwasserstoff oder 1,5-Naphthalin-disulfonsäure addiert werden.

Schliesslich wurde gefunden, dass die neuen substituierten 1-Phenyl-2-triazolyl-1-penten-3-ole der Formel (I) sowie deren Chlorwasserstoff- und 1,5-Naphthalin-disulfonsäure-Additionssalze starke pflanzenwachstumsregulierende und starke fungizide Eigenschaften aufweisen.

Überraschenderweise zeigen die erfindungsgemässen Verbindungen eine bessere pflanzenwachstumsregulierende und fungizide Wirkung als die aus dem Stand der Technik bekannten 4,4-Dimethyl-1-phenyl-2-triazolyl-1-penten-3-ole, welche chemisch und wirkungsmässig naheliegende Verbindungen sind (vgl. DE-A-2 838 847 und GB-A-2 004 276).

Die erfindungsgemässen Stoffe sind dadurch charakterisiert, dass der Phenyl-Rest mindestens einen Halogenalkoxy-, Halogenalkylthio-, Hydroxy-, Dialkylamino-, Alkylcarbonyloxy- oder Benzyloxy-Substituenten trägt. Da Stoffe mit derartigen Substituenten in der entsprechenden Position in der GB-A-2 046 260 nicht offenbart werden, unterscheiden sich die erfindungsgemässen 1-Phenyl-2-triazolyl-1-penten-3-ole der Formel (I) konstitutionell eindeutig von den strukturell ähnlichsten vorbekannten Verbindungen, denen sie auch bezüglich ihrer pflanzenwuchsregulierenden und fungiziden Wirksamkeit überraschenderweise überlegen sind.

Besonders bevorzugt sind diejenigen Verbindungen der Formel (I), in denen X für Trifluormethoxy, Trifluormethylthio, 1,1,2-Trifluor-2-chlorethoxy, 1,1,2-Trifluor-2-chlorethylthio, Hydroxy, Dimethylamino, Acetoxy, tert.-Butylcarbonyloxy oder Benzyloxy steht; Y für Fluor, Chlor, Methyl, Ethyl, Isoproyl, tert.-Butyl, Methoxy oder Isopropoxy steht; der Index m für die ganzen Zahlen 1 oder 2 steht und der Index n für die ganzen Zahlen 0, 1 oder 2 steht.

Verwendet man beispielsweise 4,4-Dimethyl-2-(1,2,4-triazol-1-yl)-1-(4-trifluormethoxyphenyl)-1-penten-3-on und Natriumborhydrid als Ausgangsstoffe, so kann der Ablauf des erfindungsgemässen Verfahrens durch das folgende Formelschema wiedergegeben werden:

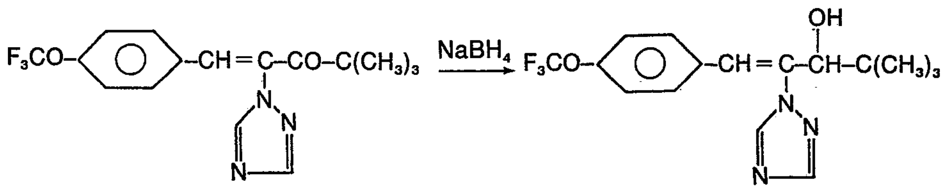


E/Z-Isomerengemisch E/Z-Isomerengemisch

Die bei der Durchführung der erfindungsgemässen Reduktion als Ausgangsstoffe benötigten 1-Phenyl-2-triazolyl-1-penten-3-one sind durch die Formel (II) allgemein definiert. In dieser Formel stehen X, Y, m und n für diejenigen Reste bzw. Zahlen, die bereits im Zusammenhang mit der Beschreibung der erfindungsgemässen Stoffe der Formel (I) für diese Substituenten bzw. Indices genannt wurden.

Die 1-Phenyl-2-triazolyl-1-penten-3-one der Formel (II) sind noch nicht bekannt. Sie können jedoch in bekannter Art und Weise erhalten werden, indem man Triazolyl-pinakolin der Formel

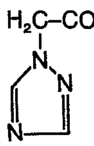

(III)

mit Aldehyden der Formel

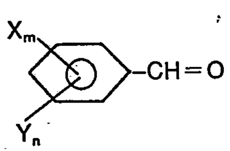

(IV)

in welcher

X, Y, m und n die oben angegebene Bedeutung haben,

in Gegenwart eines Lösungsmittels und in Gegenwart eines Katalysators umsetzt.

Als Lösungsmittel kommen für die Herstellung der 1-Phenyl-2-triazolyl-1-penten-3-one der Formel (II) vorzugsweise inerte organische Lösungsmittel in Frage. Hierzu gehören vorzugsweise Alkohole, wie Methanol und Ethanol; Ether, wie Tetrahydrofuran und Dioxan; aliphatische und cycloaliphatische Kohlenwasserstoffe, wie Benzol, Toluol und Cumol; halogenierte aliphatische und aromatische Kohlenwasserstoffe, wie Methylenchlorid, Tetrachlorkohlenstoff, Chloroform und Dichlorbenzol.

Die Herstellung der Verbindungen der Formel (II) wird in Gegenwart eines Katalysators durchgeführt. Man kann alle üblicherweise verwendbaren sauren und insbesondere basischen Katalysatoren sowie deren Puffergemische einsetzen. Hierzu gehören vorzugsweise Lewis-Säuren, wie z.B. Bortrifluorid, Bortrichlorid, Zinntetrachlorid oder Titantetrachlorid; organische Basen wie Pyridin, 2,6-Dimethylmorpholin und Piperidin; sowie insbesondere Piperidinacetat.

Die Reaktionstemperaturen können bei der Durchführung dieses Verfahrens in einem grösseren Bereich variiert werden. Im allgemeinen arbeitet man zwischen 20 und 160°C, vorzugsweise bei der Siedetemperatur des jeweiligen Lösungsmittels.

Bei der Durchführung dieses Verfahrens setzt man auf 1 Mol Triazolylpinakolin der Formel (III) 1 bis 1,5 Mol Aldehyd der Formel (IV) und katalytische bis 0,2 molare Mengen an Katalysator ein. Die Produkte der Formel (II) fallen vorzugsweise als E/Z-Isomerengemische an. Eine Trennung in die reinen Isomeren ist auf übliche Art und Weise möglich, wie z.B. durch Kristallisation oder durch chromatographische Trennverfahren.

Die 1-Phenyl-2-triazolyl-1-penten-3-one der Formel (II) stellen allgemein interessante Zwischenprodukte dar, wie z.B. zur Herstellung der erfindungsgemässen Verbindungen der Formel (I). In entsprechenden Konzentrationen zeigen sie auch wachstumsregulierende und fungizide Eigenschaften.

Die erfindungsgemässe Reduktion erfolgt in üblicher Weise, z.B. durch Umsetzung mit komplexen Hydriden, gegebenenfalls in Gegenwart eines Verdünnungsmittels, oder durch Umsetzung mit Aluminiumisopropylat in Gegenwart eines Verdünnungsmittels. Die Ausgangsstoffe der Formel (II) können dabei als E/Z-Isomerengemisch oder als reine Isomeren eingesetzt werden.

Arbeitet man mit komplexen Hydriden, so kommen als Verdünnungsmittel für die erfindungsgemässe Umsetzung polare organische Lösungsmittel in Frage. Hierzu gehören vorzugsweise Alkohole, wie Methanol, Ethanol, Butanol, Isopropanol und Ether, wie Diethylether oder Tetrahydrofuran. Die Reaktion wird im allgemeinen bei −10 bis +30°C, vorzugsweise bei −10 bis 20°C durchgeführt. Hierzu setzt man auf 1 Mol des Ketons der Formel (II) etwa 1 Mol eines komplexen Hydrids, wie Natriumborhydrid, Calciumborhydrid oder Lithiumalanat, ein. Die Isolierung der erfindungsgemässen Verbindungen erfolgt in üblicher Weise, ebenso eine eventuelle Trennung der E/Z-Isomerengemische, die bei der Reduktion mit komplexen Hydriden immer entstehen, wenn man von E/Z-Isomerengemischen als Ausgangsprodukten der Formel (II) ausgeht.

Arbeitet man mit Aluminiumisopropylat, so kommen als Verdünnungsmittel für die erfindungsgemässe Umsetzung bevorzugt Alkohole, wie Isopropanol, oder inerte Kohlenwasserstoffe, wie Benzol, in Frage. Die Reaktionstemperaturen können wiederum in einem grösseren Bereich variiert werden; im allgemeinen arbeitet man zwischen 20 und 120 °C, vorzugsweise bei 50 bis 100 °C. Zur Durchführung der Reaktion setzt man auf 1 Mol des entsprechenden Ketones der Formel (II) etwa 1 bis 2 Mol Aluminiumisopropylat ein. Die Isolierung der erfindungsgemässen Verbindungen erfolgt in üblicher Weise.

Bei der Reduktion mit Aluminiumisopropylat erhält man ausschliesslich die Z-Isomeren.

Ein eindeutiges Charakterisierungsmerkmal für die beiden geometrischen Isomeren ist die $H^1$-Kernresonanz der zwei Triazol-Protonen. Die Differenz der Shiftwerte für diese beiden Protonen ist in den E-Formen ungefähr doppelt so gross wie in den entsprechenden Z-Formen.

Die Chlorwasserstoff- und 1,5-Naphthalin-disulfonsäure-Additionssalze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure erhalten werden und in bekannter Art und Weise, z.B. durch Abfiltrieren, isoliert und gegebenenfalls durch Waschen mit einem inerten organischen Lösungsmittel gereinigt werden.

Die erfindungsgemässen Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, dass ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Strassenrändern, auf Flughäfen oder in Obstanlagen reduziert werden. Von Bedeutung ist auch die Hemmung des Wuchses von krautigen und holzigen Pflanzen an Strassenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens («Lagerns») der Pflanzen vor der Ernte verringert oder vollkommen beseitigt. Ausserdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne dass die Gefahr besteht, dass das Getreide lagert.

Eine Hemmung des vegetativen Wachstums ermöglicht bei vielen Kulturpflanzen eine dichtere Anpflanzung, so dass Mehrerträge bezogen auf die Bodenfläche erzielt werden können. Ein Vorteil der so erzielten kleineren Pflanzen ist auch, dass die Kultur leichter bearbeitet und beerntet werden kann.

Eine Hemmung des vegetativen Wachstums der Pflanzen kann auch dadurch zu Ertragssteigerungen führen, dass die Nährstoffe und Assimilate in stärkerem Masse der Blüten- und Fruchtbildung zugute kommen als den vegetativen Pflanzenteilen.

Mit Wachstumsregulatoren lässt sich häufig auch eine Förderung des vegetativen Wachstums erzielen. Dies ist von grossem Nutzen, wenn die vegetativen Pflanzenteile geerntet werden. Eine Förderung des vegetativen Wachstums kann aber auch gleichzeitig zu einer Förderung des generativen Wachstums führen, dadurch dass mehr Assimilate gebildet werden, so dass mehr oder grössere Früchte entstehen.

Ertragssteigerungen können in manchen Fällen durch einen Eingriff in den pflanzlichen Stoffwechsel erreicht werden, ohne dass sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zukker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Ausserdem lässt sich die Produktion oder der Abfluss von sekundären Pflanzeninhaltsstoffen positiv beeinflussen. Als Beispiel sei die Stimulierung des Latexflusses bei Gummibäumen genannt.

Unter dem Einfluss von Wachstumsregulatoren kann es zur Ausbildung parthenokarper Früchte kommen. Ferner kann das Geschlecht der Blüten beeinflusst werden. Auch kann eine Sterilität des Pollens erzeugt werden, was bei der Züchtung und Herstellung von Hybridsaatgut eine grosse Bedeutung hat.

Durch den Einsatz von Wachstumsregulatoren lässt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch

möglich, das Wachstum der Seitentriebe zu hemmen. Für diese Wirkung besteht z. B. grosses Interesse im Tabakanbau oder bei der Anpflanzung von Tomaten.

Unter dem Einfluss von Wachstumsregulatoren kann der Blattbestand der Pflanzen so gesteuert werden, dass ein Entblättern der Pflanzen zu einem gewünschten Zeitpunkt erreicht wird. Eine derartige Entlaubung spielt bei der mechanischen Beerntung der Baumwolle eine grosse Rolle ist aber auch in anderen Kulturen wie z. B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso lässt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Masse gefördert werden («Ausdünnung»), um die Alternanz zu brechen. Unter Alternanz versteht man die Eigenart einiger Obstarten, endogen bedingt von Jahr zu Jahr sehr unterschiedliche Erträge zu bringen. Schliesslich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren lässt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen lässt. Weiterhin können Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Voraussetzungen dafür geschaffen, dass z. B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflusst werden, so dass die Pflanzen, wie z. B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schliesslich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die erfindungsgemässen Wirkstoffe weisen auch eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemässen Wirkstoffe mit besonders gutem Erfolg zur Bekämpfung solcher Pilze eingesetzt werden, die echte Mehltauerkrankungen hervorrufen, so zur Bekämpfung von Erysiphe-Arten, wie z. B. gegen den Erreger des Gersten- bzw. des Getreidemehltaus (Erysiphe graminis); oder zur Bekämpfung von Venturia-Arten, wie z. B. gegen den Erreger des Apfelschorfs (Fusicladium dendriticum).

Hervorzuheben ist, dass die erfindungsgemässen Stoffe nicht nur eine protektive Wirkung entfalten, sondern teilweise auch systemisch wirksam sind. So gelingt es, Pflanzen gegen Pilzbefall zu schützen, wenn man die Wirkstoffe über den Boden und die Wurzel oder über das Saatgut den oberirdischen Teilen der Pflanze zuführt.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid. Als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel. Als Emulgier- und/oder schaumerzeugende Mittel kommen in Frage: z. B. nichtionogene und anionische Emulgatoren, wie Polyoxyethylen-Fettsäureester, Polyoxyethylen-Fettalkoholether, z. B. Alkylarylpolyglycol-ether, Alkylsulfonate, Alkylsulfate, Arylsulfonate sowie Eiweisshydrolysate. Als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z. B. durch Giessen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemässen Verbindungen als Pflanzenwachstumsregulatoren können die Aufwandmengen in einem grösseren Bereich variiert werden. Im allgemeinen verwendet man pro Hektar Bodenfläche 0,01 bis 50 kg, bevorzugt 0,05 bis 10 kg.

Beim Einsatz der erfindungsgemässen Stoffe als Pflanzenwachstumsregulatoren gilt, dass die Anwendung in einem bevorzugten Zeitraum vorgenommen wird, dessen genaue Abgrenzung sich nach den klimatischen und vegetativen Gegebenheiten richtet.

Auch beim Einsatz der erfindungsgemässen Stoffe als Fungizide kann die Aufwandmenge je nach Art der Applikation in einem grösseren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 Gew.-%. Bei der Saatgutbehandlung werden im allgemeinen Wirkstoffmengen von 0,001 bis 50 g je kg Saatgut, vorzugsweise 0,01 bis 10 g, benötigt. Bei der Behandlung des Bodens sind Wirkstoffkonzentrationen von 0,00001 bis 0,1 Gew.-%, vorzugsweise von 0,0001 bis 0,02%, am Wirkungsort erforderlich.

Herstellungsbeispiele

Die in der Tabelle 1 aufgeführten erfindungsgemässen Verbindungen wurden nach dem folgenden Verfahren hergestellt:

Beispiel 1

(I-1)

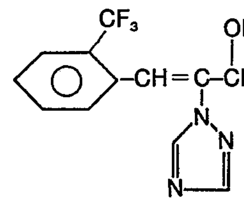

Z-Isomeres

(II-1)

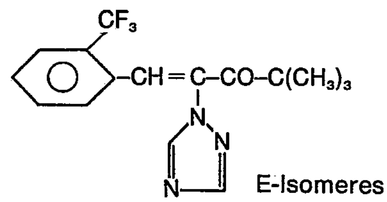

E-Isomeres

44,1 g (0,1365 Mol)
4,4-Dimethyl-2-(1,2,4-triazol-1-yl)-1-(2-trifluormethylphenyl)-1-penten-3-on
als E/Z-Isomerengemisch und 27,9 g (0,1365 Mol) Aluminiumisopropylat werden 7 Stunden in 350 ml siedendem Isopropanol erhitzt; hierbei wird über eine 30 cm-Vigreuxkolonne kontinuierlich Isopropanol und Aceton abdestilliert bis im Destillat kein Aceton mehr nachzuweisen ist. Danach wird die Lösung mit Eis/Salzsäure zersetzt. Nach Extraktion mit Methylenchlorid wird die organische Phase über Natriumsulfat getrocknet, filtriert und im Vakuum eingedampft. Die verbleibende halbkristalline Masse wird über Kieselgel-60 (Merck)/ Chloroform chromatographiert. Die ersten Fraktionen ergeben nach Abdampfen des Lösungsmittels 10,8 g (24% d. Theorie) E-isomeres
4,4-Dimethyl-2-(1,2,4-triazol-1-yl)1-(2-trifluormethylphenyl)-1-penten-3-on
vom Schmelzpunkt 82 °C.

Die nächsten Fraktionen ergeben nach Abdampfen des Lösungsmittels 12,8 g (28% der Theorie) Z-isomeres
4,4-Dimethyl-2-(1,2,4-triazol-1-yl)-1-(2-trifluormethylphenyl)-1-penten-3-ol
vom Schmelzpunkt 136 °C.

Herstellung des Ausgangsproduktes

(II–2) 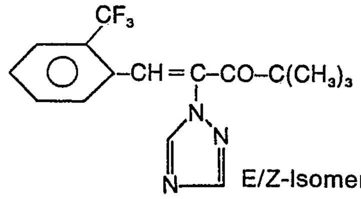 E/Z-Isomerengemisch

25,2 g (0,15 Mol) 3,3-Dimethyl-1-(1,2,4-triazol-1-yl)-butan-2-on und 26,1 g (0,15 Mol) 2-Trifluorbenzaldehyd werden in 350 ml Toluol mit 9 g Essigsäure und 3,6 ml Dimethylmorpholin 20 Stunden unter Rückfluss erhitzt, wobei das Reaktionswasser azeotrop entfernt wird. Die Toluollösung wird mit Wasser gewaschen, über Natriumsulfat getrocknet, filtriert und im Vakuum eingedampft. Man erhält 44,9 g (93% der Theorie) 4,4-Dimethyl-2-(1,2,4-triazol-1-yl)-1-(2-trifluormethylphenyl)-1-penten-3-on als E/Z-Isomerengemisch vom Brechungsindex $n_D^{20}$ = 1,5113.

In entsprechender Weise werden die folgenden Verbindungen der allgemeinen Formel (I) erhalten:

Tabelle 1

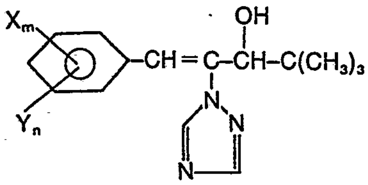 (I)

| Bsp. Nr. | $X_m$ | $Y_n$ | Schmelzpunkt (°C) |
|---|---|---|---|
| I–2 | 4–OH | – | 168 (E/Z-Gemisch) |
| I–3 | 4–OH | 3–$OCH_3$ | 136 (E/Z-Gemisch) |
| I–4 | 4-$N(CH_3)_2$ | – | 140 (E/Z-Gemisch) |
| I–5 | 4–O–$CH_2$– 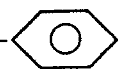 | – | 109 (E/Z-Gemisch) |
| I–6 | 4–$OCF_3$ | – | 113 (Z-Isomeres) |
| I–7 | 4–$OCF_3$ | – | 108 (E-Isomeres) |
| I–3 | 4–$SCF_3$ | – | 95 (Z-Isomeres) |
| I–9 | 4–$SCF_3$ | – | Oel (E-Isomeres) |
| I–10 | 4–$OCF_3$ | 3–Cl | 110 (Z-Isomeres) |
| I–11 | 4–$OCF_3$ | 3–Cl | 91 (E-Isomeres) |
| I–12 | 4 $CH_3COO$– | – | 130 (E-Isomeres) |
| I–13 | 2–04 | 3,5–$Cl_2$ | 158 (E/Z-Gemisch) |

Entsprechend Beispiel 1 und entsprechend dem im Text beschriebenen Verfahren werden die folgenden Ausgangsstoffe der Formel (II) erhalten:

Tabelle 2

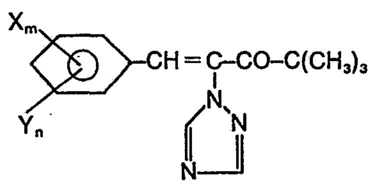 (II)

| Bsp. Nr. | $X_m$ | $Y_n$ | Schmelzpunkt (°C) bzw. Brechungsindex ($n_D^{20}$) |
|---|---|---|---|
| II–3 | 4–OH | – | 186 (E-Isomeres) |
| II–4 | 4–OH | 3–$OCH_3$ | 134 (E/Z-Gemisch) |
| II–5 | 4–$N(CH_3)_2$ | – | 112 (E/Z-Gemisch) |
| II–6 | 4–O–$CH_2$– 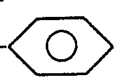 | – | 120 (E/Z-Gemisch) |
| II–7 | 4–O–$CF_3$ | – | 98 (E-Isomeres) |
| II–8 | 4–$OCF_3$ | – | 1,5145 (E/Z-Gemisch) |
| II–9 | 4–$OCF_3$ | 3–Cl | 1,5195 (E/Z-Gemisch) |
| II–10 | 4 $CH_3COO$– | – | 95 (E-Isomeres) |
| II–11 | 2–04 | 3,5–$Cl_2$ | 210 (E/Z-Gemisch) |

Anwendungsbeispiele

In den nachfolgenden Anwendungsbeispielen werden die nachstehend angegebenen Verbindungen als Vergleichssubstanzen eingesetzt:

(A) = 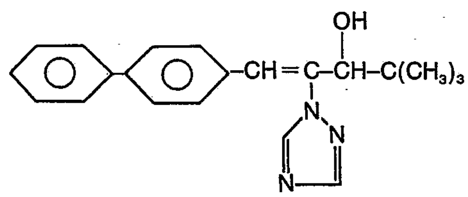

bekannt aus: DE-A 2 920 437, Verb. 3; GB-A 2 004 276, Verb. 12 (= DE-A 2 838 847)

(B) = 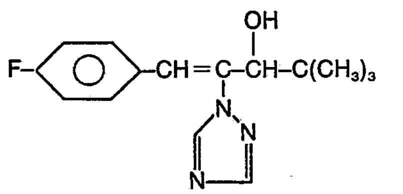

bekannt aus: DE-A 2 920 437, Verb. 2; GB-A 2 004 276, Verb. 15 (= DE-A 2 838 847)

(C) = 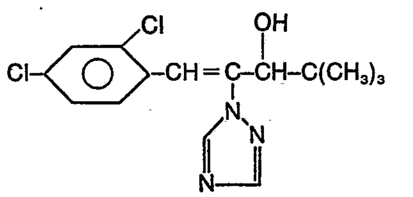

bekannt aus: GB-A 2 004 276, Verb. 14; (=DE-A 2 838 847)

Beispiel A

Wuchshemmung bei Gras (Festuca pratensis)

Lösungsmittel:

30 Gewichtsteile Dimethylformamid

Emulgator:

1 Gewichtsteil Polyoxyethylen-Sorbitan-Monolaurat

Zur Herstellung einer zweckmässigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Gras (Festuca pratensis) wird im Gewächshaus bis zu einer Wuchshöhe von 5 cm angezogen. In diesem Stadium werden die Pflanzen mit den Wirkstoffzubereitungen tropfnass besprüht. Nach 3 Wochen wird der Zuwachs gemessen und die Wuchshemmung in Prozent des Zuwachses der Kontrollpflanzen berechnet. Es bedeuten 100% Wuchshemmung den Stillstand des Wachstums und 0% ein Wachstum entsprechend dem der Kontrollpflanzen.

Der Wirkstoff gemäss Beispiel (I–7) zeigt in diesem Test eine bessere Wuchshemmung als die aus dem Stand der Technik bekannten Verbindungen (A), (B) und (C).

Beispiel B

Wuchshemmung bei Baumwolle

Lösungsmittel:

30 Gewichtsteile Dimethylformamid

Emulgator:

1 Gewichtsteil Polyoxyethylen-Sorbitan-Monolaurat

Zur Herstellung einer zweckmässigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnass mit den Wirkstoffzubereitungen besprüht. Nach 3 Wochen wird der Zuwachs der Pflanzen gemessen und die Wuchshemmung in Prozent des Zuwachses der Kontrollpflanzen berechnet. Es bedeuten 100% Wuchshemmung den Stillstand des Wachstums und 0% ein Wachstum entsprechend dem der Kontrollpflanzen.

Die Wirkstoffe gemäss Beispielen (I–13), (I–7) und (I–8) zeigen in diesem Test eine bessere Wuchshemmung als die aus dem Stand der Technik bekannten Verbindungen (A), (B) und (C).

Beispiel C

Wuchshemmung bei Sojabohnen

Lösungsmittel:

10 Gewichtsteile Dimethylformamid

Emulgator:

2 Gewichtsteile Polyoxyethylen-Sorbitan-Monolaurat

Zur Herstellung einer zweckmässigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Junge Sojabohnenpflanzen werden in dem Stadium, in dem die ersten Folgeblätter entfaltet sind, mit den Wirkstoffzubereitungen tropfnass besprüht. Nach 2 Wochen wird der Zuwachs gemessen und die Wuchshemmung in % des Zuwachses der Kontrollpflanzen berechnet. Es bedeuten 100% den Stillstand des Wachstums und 0% ein Wachstum entsprechend dem der unbehandelten Kontrollpflanzen.

Die Wirkstoffe gemäss Beispielen (I–7) und (I–8) zeigen in diesem Test eine bessere Wuchshemmung als die aus dem Stand der Technik bekannte Verbindung (A).

Beispiel D

Wuchshemmung bei Zuckerrüben

Lösungsmittel:

30 Gewichtsteile Dimethylformamid

Emulgator:

1 Gewichtsteil Polyoxyethylen-Sorbitan-Monolaurat

Zur Herstellung einer zweckmässigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Zuckerrüben werden im Gewächshaus bis zur vollen Ausbildung der Keimblätter angezogen. In diesem Stadium werden die Pflanzen tropfnass mit den Wirkstoffzubereitungen besprüht. Nach 14 Tagen wird der Zuwachs der Pflanzen gemessen und die Wuchsbeeinflussung in Prozent des Zuwachses der Kontrollpflanzen berechnet. Es bedeuten 0% Wuchshemmung ein Wachstum entsprechend dem der Kontrollpflanzen. 100% Wuchshemmung bedeutet Stillstand des Wachstums.

Die Wirkstoffe gemäss Beispielen (I–7) und (I–8) zeigen in diesem Test eine bessere Wuchshemmung als die aus dem Stand der Technik bekannten Verbindungen (A) und (B).

Beispiel E

Stimulierung der Photosynthese

Nach Behandlung von Sojabohnenpflanzen mit den Wirkstoffen gemäss Herstellungsbeispielen (I–6) und (I–7) wurde eine Stimulierung der Photosynthese gegenüber Kontrollpflanzen festgestellt. Demgegenüber brachte die Behandlung mit der bekannten Verbindung (A) diesen Effekt nicht.

Beispiel F

Erysiphe-Test (Gerste)/protektiv

Lösungsmittel:

100 Gewichtsteile Dimethylformamid

Emulgator:

0,25 Gewichtsteile Alkyl-aryl-polyglykolether

Zur Herstellung einer zweckmässigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20 °C und einer relativen Luftfeuchtigkeit von ca. 80% aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen in diesem Test z.B. die Verbindungen gemäss folgender Herstellungsbeispiele: (I–7) und (I–8).

**Patentansprüche**

1. Substituierte 1-Phenyl-2-triazolyl-1-penten-3-ole der Formel

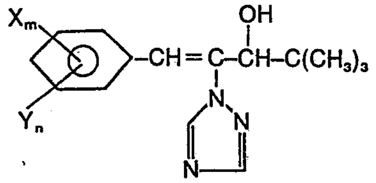

(I)

in welcher

X für Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Hydroxy, Dialkylamino mit 1 bis 4 Kohlenstoffatomen in jedem Alkylteil, Alkylcarbonyloxy mit 1 bis 4 Kohlenstoffatomen im Alkylteil oder für Benzyloxy steht,

Y für Fluor, Chlor, Brom, Alkyl mit 1 bis 4 Kohlenstoffatomen oder Alkoxy mit 1 bis 4 Kohlenstoffatomen steht,

m für 1 oder 2 steht und

n für 0, 1 oder 2 steht

sowie deren 1,5-Naphthalin-disulfonsäure- oder Chlorwasserstoff-Additionssalze.

2. Verfahren zur Herstellung von substituierten 1-Phenyl-2-triazolyl-1-penten-3-olen der Formel

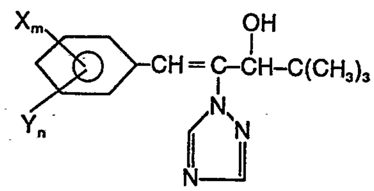

(I)

in welcher

X für Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Hydroxy, Dialkylamino mit 1 bis 4 Kohlenstoffatomen in jedem Alkylteil, Alkylcarbonyloxy mit 1 bis 4 Kohlenstoffatomen im Alkylteil oder für Benzyloxy steht,

Y für Fluor, Chlor, Brom, Alkyl mit 1 bis 4 Kohlenstoffatomen oder Alkoxy mit 1 bis 4 Kohlenstoffatomen steht,

m für 1 oder 2 steht und

n für 0, 1 oder 2 steht

sowie von deren 1,5-Naphthalin-disulfonsäure- oder Chlorwasserstoff-Additionssalzen, dadurch gekennzeichnet, dass man 1-Phenyl-2-triazolyl-1-penten-3-one der Formel

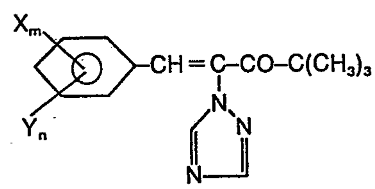

(II)

in welcher

X, Y, n und m die oben angegebene Bedeutung haben, in allgemein üblicher Weise reduziert und gegebenenfalls anschliessend an die erhaltenen Verbindungen der Formel (I) 1,5-Naphthalin-disulfonsäure oder Chlorwasserstoff addiert.

3. Pflanzenwachstumsregulierende und fungizide Mittel, gekennzeichnet durch einen Gehalt an mindestens einem substituierten 1-Phenyl-2-triazolyl-1-penten-3-ol der Formel (I) gemäss Anspruch 1 bzw. einem 1,5-Naphthalin-disulfonsäure- oder Chlorwasserstoff-Additionssalz eines substituierten 1-Phenyl-2-triazolyl-1-penten-3-ols der Formel (I) gemäss Anspruch 1.

4. Verfahren zur Regulierung des Pflanzenwachstums sowie zur Bekämpfung von Pilzen, dadurch gekennzeichnet, dass man substituierte 1-Phenyl-2-triazolyl-1-penten-3-ole der Formel (I) gemäss Anspruch 1 bzw. deren 1,5-Naphthalin-disulfonsäure oder Chlorwasserstoff-Additionssalze auf die Pflanzen und/oder deren Lebensraum ausbringt.

5. Verwendung von substituierten 1-Phenyl-2-triazolyl-1-penten-3-olen der Formel (I) gemäss Anspruch 1 bzw. von 1,5-Naphthalin-disulfonsäure oder Chlorwasserstoff-Additionssalzen von substituierten 1-Phenyl-2-triazolyl-1-penten-3-olen der Formel (I) gemäss Anspruch 1 zur Regulierung des Pflanzenwachstums bzw. zur Bekämpfung von Pilzen.

6. Verfahren zur Herstellung von pflanzenwachstumsregulierenden und fungiziden Mitteln, dadurch gekennzeichnet, dass man substituierte 1-Phenyl-2-triazolyl-1-penten-3-ole der Formel (I) gemäss Anspruch 1 bzw. 1,5-Naphthalin-disulfonsäure- oder Chlorwasserstoff-Additionssalze von substituierten 1-Phenyl-2-triazolyl-1-penten-3-olen der Formel (I) gemäss Anspruch 1 mit Streckmitteln und/oder oberflächenaktiven Stoffen vermischt.

7. 1-Phenyl-2-triazolyl-1-penten-3-one der Formel

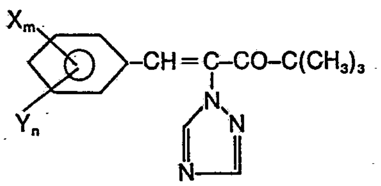

(II)

in welcher

X für Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Hydroxy, Dialkylamino mit 1 bis 4 Kohlenstoffatomen in jedem Alkylteil, Alkylcarbonyloxy mit 1 bis 4 Kohlenstoffatomen im Alkylteil oder für Benzyloxy steht,

Y für Fluor, Chlor, Brom, Alkyl mit 1 bis 4 Kohlenstoffatomen oder Alkoxy mit 1 bis 4 Kohlenstoffatomen steht,

m für 1 oder 2 steht und

n für 0, 1 oder 2 steht.

8. Verfahren zur Herstellung von 1-Phenyl-2-triazolyl-1-penten-3-onen der Formel

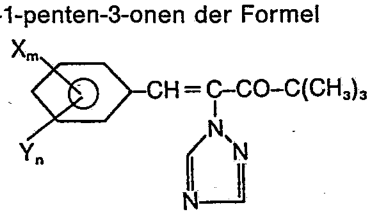

(II)

in welcher

X für Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Hydroxy, Dialkylamino mit 1 bis 4 Kohlenstoffatomen in jedem Alkylteil, Alkylcarbonyloxy mit 1 bis 4 Kohlenstoffatomen im Alkylteil oder für Benzyloxy steht,

Y für Fluor, Chlor, Brom, Alkyl mit 1 bis 4 Kohlenstoffatomen oder Alkoxy mit 1 bis 4 Kohlenstoffatomen steht,

m für 1 oder 2 steht und

n für 0, 1 oder 2 steht,

dadurch gekennzeichnet, dass man Triazolylpinakolin der Formel

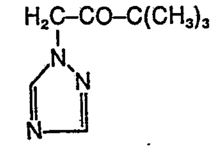

(III)

mit Aldehyden der Formel

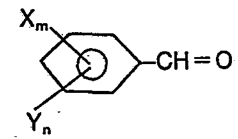

(IV)

in welcher

X, Y, m und n die oben angegebene Bedeutung haben,

in Gegenwart eines Lösungsmittels und in Gegenwart eines Katalysators umsetzt.

**Claims**

1. Substituted 1-phenyl-2-triazolyl-1-penten-3-ols of the formula

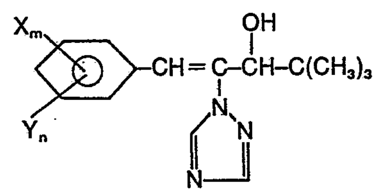

(I)

in which

X represents halogenoalkoxy with 1 or 2 carbon atoms and 1 to 5 identical or different halogen atoms, halogenoalkylthio with 1 or 2 carbon atoms and 1 to 5 identical or different halogen atoms, hydroxyl, dialkylamino with 1 to 4 carbon atoms in each alkyl part, alkylcarbonyloxy with 1 to 4 carbon atoms in the alkyl part or benzyloxy,

Y represents fluorine, chlorine, bromine, alkyl with 1 to 4 carbon atoms or alkoxy with 1 to 4 carbon atoms,

m represents 1 or 2 and

n represents 0, 1 or 2,

and 1,5-naphthalene-disulphonic acid or hydrogen chloride addition salts thereof.

2. Process for the preparation of substituted 1-phenyl-2-triazolyl-1-penten-3-ols of the formula

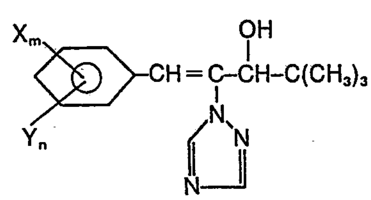

(I)

in which

X represents halogenoalkoxy with 1 or 2 carbon atoms and 1 to 5 identical or different halogen atoms, halogenoalkylthio with 1 or 2 carbon atoms and 1 to 5 identical or different halogen atoms, hydroxyl, dialkylamino with 1 to 4 carbon atoms in each alkyl part, alkylcarbonyloxy with 1 to 4 carbon atoms in the alkyl part or benzyloxy,

Y represents fluorine, chlorine, bromine, alkyl with 1 to 4 carbon atoms or alkoxy with 1 to 4 carbon atoms,

m represents 1 or 2 and

n represents 0, 1 or 2,

and 1,5-naphthalene-disulphonic acid or hydrogen chloride addition salts thereof, characterised in that 1-phenyl-2-triazolyl-1-penten-3-ones of the formula

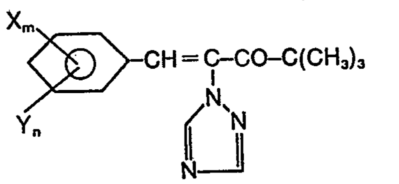

(II)

in which

X, Y, n and m have the abovementioned meaning,

are reduced in a generally customary manner and, if desired, 1,5-naphthalene-disulphonic acid or hydrogen chloride is then added on to the compounds of the formula (I) obtained.

3. Plant-growth-regulating and fungicidal agents, characterised in that they contain at least one substituted 1-phenyl-2-triazolyl-1-penten-3-ol of the formula (I) according to Claim 1 or at least one 1,5-naphthalene-disulphonic acid or hydrogen chloride addition salt of a substituted 1-phenyl-2-triazolyl-1-penten-3-ol of the formula (I) according to Claim 1.

4. Method of regulating plant growth and of combating fungi, characterised in that substituted 1-phenyl-2-triazolyl-1-penten-3-ols of the formula (I) according to Claim 1 or 1,5-naphthalene-disulphonic acid or hydrogen chloride addition salts thereof are applied to the plants and/or their environment.

5. Use of substituted 1-phenyl-2-triazolyl-1-penten-3-ols of the formula (I) according to Claim 1 or 1,5-naphthalene-disulphonic acid or hydrogen chloride addition salts of substituted 1-phenyl-2-triazolyl-1-penten-3-ols of the formula (I) according to Claim 1 for regulating plant growth or for combating fungi.

6. Process for the preparation of plant-growth-regulating and fungicidal agents, characterised in that substituted 1-phenyl-2-triazolyl-1-penten-3-ols of the formula (I) according to Claim 1 or 1,5-naphthalene-disulphonic acid or hydrogen chloride addition salts of substituted 1-phenyl-2-triazolyl-1-penten-3-ols of the formula (I) according to Claim 1 are mixed with extenders and/or surface-active substances.

7. 1-Phenyl-2-triazolyl-1-penten-3-ones of the formula

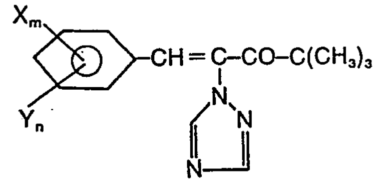

(II)

in which

X represents halogenoalkoxy with 1 or 2 carbon atoms and 1 to 5 identical or different halogen atoms, halogenoalkylthio with 1 or 2 carbon atoms and 1 to 5 identical or different halogen atoms, hydroxyl, dialkylamino with 1 to 4 carbon atoms in each alkyl part, alkylcarbonyloxy with 1 to 4 carbon atoms in the alkyl part or benzyloxy,

Y represents fluorine, chlorine, bromine, alkyl with 1 to 4 carbon atoms or alkoxy with 1 to 4 carbon atoms,

m represents 1 or 2 and

n represents 0, 1 or 2.

8. Process for the preparation of 1-phenyl-2-triazolyl-1-penten-3-ones of the formula

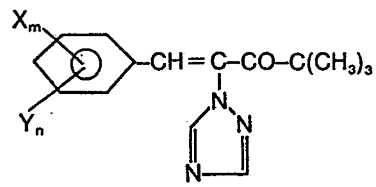

(II)

in which

X represents halogenoalkoxy with 1 or 2 carbon atoms and 1 to 5 identical or different halogen atoms, halogenoalkylthio with 1 or 2 carbon atoms and 1 to 5 identical or different halogen atoms, hydroxyl, dialkylamino with 1 to 4 carbon atoms in each alkyl part, alkylcarbonyloxy with 1 to 4 carbon atoms in the alkyl part or benzyloxy,

Y represents fluorine, chlorine, bromine, alkyl with 1 to 4 carbon atoms or alkoxy with 1 to 4 carbon atoms,

m represents 1 or 2 and

n represents 0, 1 or 2,

characterised in that triazolylpinacoline of the formula

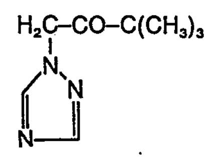

(III)

is reacted with aldehydes of the formula

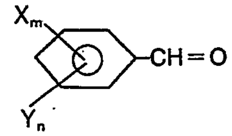

(IV)

in which

X, Y, m and n have the abovementioned meaning,

in the presence of a solvent and in the presence of a catalyst.

**Revendications**

1. 1-Phényl-2-triazolyl-1-pentène-3-ols substitués de formule

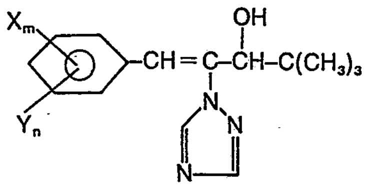

(I)

dans laquelle

X est un groupe halogénalkoxy ayant 1 ou 2 atomes de carbone et 1 à 5 atomes d'halogènes égaux ou différents, un groupe halogénalkylthio ayant 1 ou 2 atomes de carbone et 1 à 5 atomes d'halogènes égaux ou différents, un groupe hydroxy, dialkylamino ayant 1 à 4 atomes de carbone dans chaque partie alkyle, un groupe alkylcarbonyloxy ayant 1 à 4 atomes de carbone dans la partie alkyle ou le groupe benzyloxy,

Y désigne le fluor, le chlore, le brome, un groupe alkyle ayant 1 à 4 atomes de carbone ou un groupe alkoxy ayant 1 à 4 atomes de carbone,

m a la valeur 1 ou 2 et

n a la valeur 0, 1 ou 2,

ainsi que leurs sels d'addition d'acide 1,5-naphtalène-disulfonique ou de chlorure d'hydrogène.

2. Procédé de production de 1-phényl-2-triazolyl-1-pentène-3-ols substitués de formule

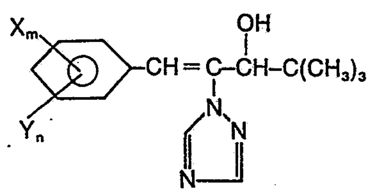

(I)

dans laquelle

X est un groupe halogénalkoxy ayant 1 ou 2 atomes de carbone et 1 à 5 atomes d'halogènes égaux ou différents, un groupe halogénalkylthio ayant 1 ou 2 atomes de carbone et 1 à 5 atomes d'halogènes égaux ou différents, un groupe hydroxy, dialkylamino ayant 1 à 4 atomes de carbone dans chaque partie alkyle, un groupe alkylcarbonyloxy ayant 1 à 4 atomes de carbone dans la partie alkyle ou le groupe benzyloxy,

Y désigne le fluor, le chlore, le brome, un groupe alkyle ayant 1 à 4 atomes de carbone ou un groupe alkoxy ayant 1 à 4 atomes de carbone,

m a la valeur 1 ou 2 et

n a la valeur 0, 1 ou 2,

ainsi que de leurs sels d'addition d'acide 1,5-naphtalène-disulfonique ou de chlorure d'hydrogène, caractérisé en ce qu'on réduit d'une manière généralement classique des 1-phényl-2-triazolyl-1-pentène-3-ones de formule

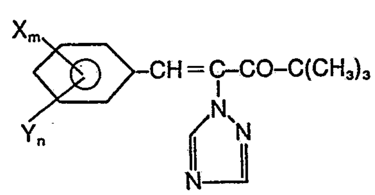

(II)

dans laquelle X, Y, m et n ont les définitions données ci-dessus, puis on additionne le cas échéant sur les composés de formule (I) obentus l'acide 1,5-naphtalène-disulfonique ou du chlorure d'hydrogène.

3. Compositions influençant la croissance de végétaux et fongicides, caractérisées en une teneur en au moins un 1-phényl-2-triazolyl-1-pentène-3-ol substitué de formule (I) suivant la revendication 1 ou en un sel d'addition d'acide 1,5-naphtalène-disulfonique ou de chlorure d'hydrogène d'un 1-phényl-2-triazolyl-1-pentène-3-ol substitué de formule (I) suivant la revendication 1.

4. Procédé pour influencer la croissance de végétaux ainsi que pour combattre des champignons, caractérisé en ce qu'on applique des 1-phényl-2-triazolyl-1-pentène-3-ols substitués de formule (I) suivant la revendication 1 ou leurs sels d'addition d'acide 1,5-naphtalène-disulfonique ou de chlorure d'hydrogène sur les végétaux et/ou sur leur milieu.

5. Utilisation de 1-phényl-2-triazolyl-1-pentène-3-ols substitués de formule (I) suivant la revendication 1 ou de sels d'addition d'acide 1,5-naphtalène-disulfonique ou de chlorure d'hydrogène de 1-phényl-2-triazolyl-1-pentène-3-ols substitués de formule (I) suivant la revendication 1 pour influencer la croissance des végétaux ou pour combattre des champignons.

6. Procédé de préparation de compositions influençant la croissance de végétaux et fongicides, caractérisé en ce qu'on mélange des 1-phényl-2-triazolyl-1-pentène-3-ols substitués de formule (I) suivant la revendication 1 ou des sels d'addition d'acide 1,5-naphtalène-disulfonique ou de chlorure d'hydrogène de 1-phényl-2-triazolyl-1-pentène-3-ols substitués de formule (I) suivant la revendication 1 avec des diluants et/ou des agents tensio-actifs.

7. 1-Phényl-2-triazolyl-1-pentène-3-ones de formule

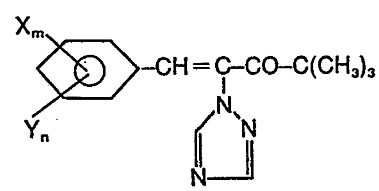

(II)

dans laquelle

X est un groupe halogénalkoxy ayant 1 ou 2 atomes de carbone et 1 à 5 atomes d'halogènes égaux ou différents, halogénalkylthio ayant 1 ou 2 atomes de carbone et 1 à 5 atomes d'halogènes égaux ou différents, hydroxy, dialkylamino ayant 1 à 4 atomes de carbone dans chaque partie alkyle, alkylcarbonyloxy ayant 1 à 4 atomes de carbone dans la partie alkyle ou benzyloxy,

Y désigne le fluor, le chlore, le brome, un groupe alkyle ayant 1 à 4 atomes de carbone, ou alkoxy ayant 1 à 4 atomes de carbone,

m a la valeur 1 ou 2 et

n a la valeur 0, 1 ou 2.

8. Procédé de production de 1-phényl-2-triazolyl-1-pentène-3-ones de formule

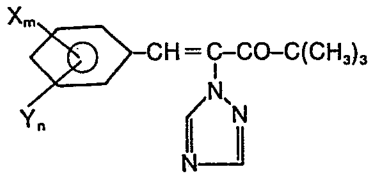

(II)

dans laquelle

X est un groupe halogénalkoxy ayant 1 ou 2 atomes de carbone et 1 à 5 atomes d'halogènes égaux ou différents, halogénalkylthio ayant 1 ou 2 atomes de carbone et de 1 à 5 atomes d'halogènes égaux ou différents, hydroxy, dialkylamino ayant 1 à 4 atomes de carbone dans chaque partie alkyle, alkylcarbonyloxy ayant 1 à 4 atomes de carbone dans la partie alkyle ou benzyloxy,

Y désigne le fluor, le chlore, le brome, un groupe alkyle ayant 1 à 4 atomes de carbone ou alkoxy ayant 1 à 4 atomes de carbone,

m a la valeur 1 ou 2 et

n a la valeur 0, 1 ou 2,

caractérisé en ce qu'on fait réagir la triazolylpinacoline de formule

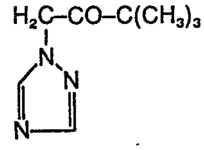

(III)

avec des aldéhydes de formule

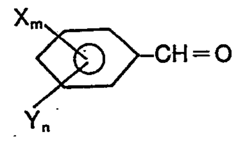

(IV)

dans laquelle

X, Y, m et n ont la définition indiquée ci-dessus, en présence d'un solvant et en présence d'un catalyseur.